# EUROPEAN PATENT APPLICATION

(11) **EP 4 563 138 A1**
(43) Date of publication of application: **04.06.2025**
(21) Application number: 23846392.1
(22) Date of filing: 21.07.2023
(51) Int. Cl.: A61K 6/836, A61K 6/802

(54) **PORCELAIN PASTE FOR DENTISTRY**

(30) Priority: 25.07.2022 JP 2022117947
(71) Applicant: Kuraray Noritake Dental Inc., Kurashiki-shi, Okayama 710-0801 (JP)
(72) Inventor: TAKEUCHI, Yuta, Miyoshi-shi, Aichi 470-0293 (JP)
(74) Representative: D Young & Co LLP
(86) International application number: PCT/JP2023/026743
(87) International publication number: WO 2024/024661

(57) **Abstract**

The present invention relates to a dental porcelain paste containing a cationic surfactant (A), glass powder (B), and an alcohol (C).

## Description

### Technical Field

The present invention relates to a dental porcelain paste.

### Background Art

Ceramics have transparency and color similar to those of natural teeth, and therefore are being an indispensable material for producing dental crowns, which require aesthetic quality.

In general, a dental crown is produced by repeating an operation of building up a dental admixture containing a porcelain material, one or more kinds selected from water and an organic solvent, and the like, or a porcelain material in the form of paste formed by mixing one or more kinds selected from water and an organic solvent, and the like with ceramics in advance, on a frame (such as a metal frame or a ceramic frame) covering the abutment tooth, and then baking.

A porcelain material in the form of powder requires a complicated procedure of mixing with a solvent every time performing the operation, and requires a skilled operation for regulating the mixing ratio corresponding to the desired color tone, and the viscosity thereof, but the porcelain material in the form of paste contains a porcelain material and a solvent that have been mixed to have a viscosity enabling the operation by the dental technician with good handleability, and therefore can reduce the period of time of dental technician work.

For example, PLT 1 describes a dental paste porcelain material that contains a component containing a synthetic and/or natural polymer material having a hydrophilic group, dissolved in one kind or two or more kinds of an organic solvent selected from a dihydric or trihydric alcohol, an ether having a hydroxy group remaining therein, and a hydroxy(meth)acrylate, and/or water, and describes that the dental paste porcelain material is stable in long-term storage and is not easily dried or hardened during use.

PTL 2 describes a dental paste material that contains a dental porcelain material and a solvent, to which 0.1 to 20% by weight of inorganic powder having an average particle diameter of 5 to 50 nm, and describes that the dental porcelain material is not settled in long-term storage, resulting in excellent workability.

PTL 3 describes a dental paste porcelain material that contains hydrophobic fine particle silica having a particular average primary particle diameter, and contains glass powder having a regulated particle diameter and an organic solvent having a particular boiling point in particular amounts, and describes that a dental porcelain paste can retain the paste state for a long time, has excellent coatability, and is substantially free of carbonization and bubbles caused by the organic component or the polymer component in baking.

### Citation List

### Patent Literatures

PTL 1: JP2001-079019A
PTL 2: JPH3-077804A
PTL 3: JP2020-117499A

### Summary of Invention

### Technical Problem

However, in the dental paste porcelain material described in PTL 1, it has been found that the porcelain material is discolored to black or gray in baking the porcelain material, and there is room for improvement in aesthetic quality.

In the dental paste material described in PTL 2, the dental paste material is colored with the inorganic fine powder, resulting in concern that the aesthetic quality is deteriorated.

In the dental paste described in PTL 3, it has been found that the glass powder and the solvent are separated from each other in long-term storage, and the solidified sedimentation layer cannot be mixed, making it difficult to restore the coatability immediately after producing the dental paste.

Under the circumstances, an object of the present invention is to provide a dental porcelain paste that can be suppressed in discoloration and reduction in transparency after baking, is not solidified even in long-term storage, and can easily restore the state immediately after producing the dental porcelain paste.

### Solution to Problem

As a result of earnest investigations by the present inventors, it has been found that the problem can be solved by using a dental porcelain paste containing a cationic surfactant (A), glass powder (B), and an alcohol (C), and thus the present invention has been completed.

Specifically, the present invention encompasses the following inventions.
[1] A dental porcelain paste containing a cationic surfactant (A), glass powder (B), and an alcohol (C).
[2] The dental porcelain paste according to the item [1], in which the dental porcelain paste has a content of the component (A) of 0.0005 to 2.0 parts by mass per 100 parts by mass of the component (B).
[3] The dental porcelain paste according to the item [1] or [2], in which the dental porcelain paste has a content of the component (C) of 20 to 60 parts by mass per 100 parts by mass in total of the component (B) and the component (C).
[4] The dental porcelain paste according to any one of the items [1] to [3], in which the dental porcelain paste has a total content of the component (A), the component (B), and the component (C) of 75% by mass or more based on the dental porcelain paste as 100% by mass.
[5] The dental porcelain paste according to any one of the items [1] to [4], in which the component (C) contains a dihydric alcohol.
[6] The dental porcelain paste according to any one of the items [1] to [5], in which the component (B) has an average particle diameter of 1 to 30 µm.
[7] The dental porcelain paste according to any one of the items [1] to [6], in which a residue on ignition of the component (A) is 8.0% or less.

### Advantageous Effects of Invention

The present invention can provide a dental porcelain paste that can be suppressed in discoloration and reduction in transparency after baking, is not solidified even in long-term storage, and can easily restore the state immediately after producing the dental porcelain paste.

### Description of Embodiments

The present invention will be described in detail with reference to embodiments below.

In the description herein, the upper limit values and the lower limit values of the numeral range (such as the content of the component, the value calculated from the components, and the property value) can be appropriately combined.

Therefore, in the description herein, the lower limit values and the upper limit values described in a stepwise manner for the numeral range can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value, and similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as "10 or more" or "30 or more", and similarly, only the upper limit value can be defined as "90 or less" or "60 or less".

In the description herein, unless otherwise indicated, the term "restorability" means such characteristics that the dental porcelain paste is not solidified in long-term storage, and can easily restore the state immediately after producing the dental porcelain paste through agitation or the like, and specifically means characteristics that are evaluated by the method described in the examples.

In the description herein, unless otherwise indicated, the term "appearance" means "discoloration" and "transparency" in baking the dental porcelain paste, and specifically means characteristics that are evaluated by the method described in the examples.

### [Dental Porcelain Paste]

The dental porcelain paste as one embodiment of the present invention contains a cationic surfactant (A) (which may be hereinafter referred simply to as a "component (A)"), glass powder (B) (which may be hereinafter referred simply to as a "component (B)"), and an alcohol (C) (which may be hereinafter referred simply to as a "component (C)").

The dental porcelain paste can exert the effects described above with the components (A) to (C) contained. The details of the mechanism that the effects of the present invention are exerted are unclear, but can be considered as follows.

The hydrophilic group of the cationic surfactant (A) is readily adsorbed to the surface of the glass powder (B) which has a large content of silica and is negatively charged, and thereby the surface of the glass powder (B) having the cationic surfactant (A) adsorbed thereto is hydrophobized. It is estimated that the particles of the hydrophobized glass powder (B) form a loose aggregate in a hydrophilic liquid, such as the alcohol (C), and therefore even in the case where a sedimentation layer is generated in long-term storage, the sedimentation layer is not solidified, but at the time when the dental porcelain paste stored for a long time is reused, can be easily mixed through agitation or the like to restore the state immediately after producing the dental porcelain paste.

It is also estimated that the cationic surfactant (A) is adsorbed to the surface of the glass powder (B), but is not bonded thereto, and therefore an organic component derived from the cationic surfactant (A) does not remain on the surface of the glass powder (B) after baking, suppressing the formation of carbonization and bubbles in baking, by which the discoloration and reduction in transparency after baking can be suppressed.

The components contained in the dental porcelain paste as one embodiment of the present invention are described below.

### <Cationic Surfactant (A)>

The cationic surfactant (A) is not limited in kind, as long as exerting the effects of the present invention, and examples thereof include an ammonium salt having an alkyl group, such as an alkyltrimethylammonium salt, a dialkyldimethylammonium salt, an alkylbenzyldimethylammonium salt, an alkylammonium salt, and an alkylpyridinium salt. The alkyl group is preferably an alkyl group having 8 to 18 carbon atoms, and examples of the component (A) of this type include an alkyl(having 8 to 18 carbon atoms)trimethylammonium salt, a dialkyl(having 8 to 18 carbon atoms)dimethylammonium salt, an alkyl(having 8 to 18 carbon atoms)benzyldimethylammonium salt, and an alkyl(having 8 to 18 carbon atoms)pyridinium salt.

The alkyl group having 8 to 18 carbon atoms is more preferably an alkyl group having 8 to 16 carbon atoms, and further preferably an alkyl group having 8 to 14 carbon atoms, from the standpoint of the decomposability.

One kind of the component (A) may be used alone, or two or more kinds thereof may be appropriately used in combination.

The anion constituting the salt is not limited in kind, and examples thereof include an ammonium ion having an alkyl group capable of forming the salt described above, such as an alkyltrimethylammonium ion, a dialkyldimethylammonium ion, an alkylbenzyldimethylammonium ion, an alkylammonium ion, and an alkylpyridinium ion.

Examples of the anion constituting the salt include a chloride ion (Cl⁻) and a bromide ion (Br⁻). Assuming the same cation constituting the salt, the anion is preferably a chloride ion from the standpoint of the decomposability.

Preferred examples of the component (A) including the combination of the anion and the cation described above include a chloride, such as cetylpyridinium chloride, lauryltrimethylammonium chloride, dodecylpyridinium chloride, trimethylstearylammonium chloride, dimethyldioctadecylammonium chloride, and benzyldimethyltetradecylammonium chloride; and a bromide, such as lauryltrimethylammonium bromide and trimethylstearylammonium bromide. Among these, at least one kind selected from the group consisting of cetylpyridinium chloride, lauryltrimethylammonium chloride, dodecylpyridinium chloride, and lauryltrimethylammonium bromide is preferred.

The chloride described above is preferred from the standpoint of the decomposability and the availability.

The component (A) preferably has good decomposability in baking, and the decomposability in baking can be evaluated, for example, by measuring the residue on ignition (i.e., the residual ratio after baking at 400°C for 1 hour).

The residue on ignition of the component (A) is preferably 8.0% or less, more preferably 5.0% or less, further preferably 1.0% or less, and still further preferably 0.5% or less. The residue on ignition of the component (A) can be measured by the method described in the examples. In other words, in one embodiment of the component (A), the residue on ignition of the component (A) is preferably 0.0 to 8.0%, more preferably 0.0 to 5.0%, further preferably 0.0 to 1.0%, and still further preferably 0.0 to 0.5%.

The content of the component (A) is preferably 0.0005 to 2.0 parts by mass per 100 parts by mass of the component (B). The content of the component (A) that is 0.0005 part by mass or more per 100 parts by mass of the component (B) is preferred since the easiness in agitation of the dental porcelain paste after long-term storage can be further easily enhanced, thereby enhancing the restorability. The content of the component (A) that is 2.0 parts by mass or less per 100 parts by mass of the component (B) is preferred since the discoloration of glass in baking the dental porcelain paste can be easily suppressed. From these standpoints, the content of the component (A) is more preferably 0.001 to 1.5 parts by mass, further preferably 0.003 to 1.2 parts by mass, still further preferably 0.008 to 0.8 part by mass, still more further preferably 0.01 to 0.5 part by mass, and even further preferably 0.05 to 0.3 part by mass, per 100 parts by mass of the component (B).

The component (A) is thermally decomposed more easily than a polymer material, and with a small amount thereof added, can easily provide the effect of preventing the solidification of the sedimentation layer generating in long-term storage of the dental porcelain paste, as described above. Therefore, it is estimated that in addition to the restorability of the dental porcelain paste after long-term storage, the formation of carbonization and bubbles in baking the dental porcelain paste can be easily suppressed, by which the deterioration of the appearance after baking can be further suppressed. Furthermore, as described above, the component (A) is adsorbed to the surface of the component (B), but is not bonded directly thereto, and therefore it is estimated that one of the factors of suppressing the deterioration in appearance is that an organic matter derived from the component (A) is unlikely to remain on the surface of glass after baking.

### <Glass Powder (B)>

The glass powder (B) is not particularly limited, may be a material capable of being applied to the dental use, and may contain crystals.

Examples of the material of the glass powder (B) include glass containing SiO₂ as a major component (i.e., a component having the largest content in the glass) and crystallized glass. The glass may contain Al₂O₃, B₂O₃, ZnO, K₂O, Na₂O, Li₂O, ZrO₂, CaO, MgO, Sb₂O₃, CeO₂, BaO, SnO₂, and the like in addition to SiO₂, and for example, at least one kind selected from the group consisting of amorphous type potassium aluminosilicate glass (4SiO₂·Al₂O₃·K₂O), leucite crystal type potassium aluminosilicate glass, and fluorapatite glass can be preferably used. Among these, amorphous type potassium aluminosilicate glass is more preferred. Examples of the crystal include leucite, potassium feldspar, fluorphlogopite, diopside, mica, β-spodumene (LiAlSi₂O₆), calcium β-metaphosphate, apatite, magnesium titanate, β- eucryptite, and alumina.

One kind of the glass powder (B) may be used alone, or two or more kinds thereof may be appropriately used in combination, depending on the purpose, such as a porcelain material fused to metal, a full ceramic prosthesis, a laminate veneer, and the like.

The raw material substance of the glass to be the material of the glass powder (B) may be ceramic materials having been ordinarily widely used, and examples thereof used include an oxide, such as SiO₂, Al₂O₃, B₂O₃, ZnO, K₂O, Na₂O, Li₂O, ZrO₂, CaO, MgO, Sb₂O₃, CeO₂, BaO, and SnO₂; a substance capable of becoming the oxide, such as SiO₂, Al₂O₃, B₂O₃, ZnO, K₂O, Na₂O, Li₂O, ZrO₂, CaO, MgO, Sb₂O₃, CeO₂, BaO, and SnO₂, in heating in the air; and a mixture of the oxide and the substance capable of becoming the oxide. In this case, the glass composition to be obtained is calculated in advance, and the formulation is determined thereby, according to which the raw material substances are mixed. The method of mixing the raw material substances is not particularly limited, and the raw material substances are preferably dispersed uniformly.

The mixed raw material substances are heated to approximately 700°C or more. The method of heating is not particularly limited, and it suffices that the raw material substances are completely melted to form a uniform molten matter. The method of cooling the molten matter is also not particularly limited, and may be air cooling or the like.

The glass gob obtained in this manner is pulverized and classified to provide the glass powder (B) having a regulated particle size. The method of pulverizing and classifying the glass gob is not particularly limited, and examples of the pulverizer include a compression pulverizer, such as a jaw crusher and a cone crusher; a ball mill, such as a vibration ball mill and a planetary ball mill; a medium agitation type pulverizer, such as a tower pulverizer, an agitation tank pulverizer, and an annular type pulverizer; and a high-speed rotation impact pulverizer, such as a pin mill and a disk mill; and also include a roll mill, a jet pulverizer, and an autogenous pulverizer. Examples of the classifier include a sieve classifier, such as a vibration sieve and a shifter; a centrifugal classifier, such as a cyclone; and a wet classifier, such as a sedimentation classifier. The pulverizer and the classifier each are preferably a device coated with a resin or glass for avoiding metal impurities.

The average particle diameter of the component (B) is preferably 1 to 30 µm, more preferably 2 to 20 µm, and further preferably 3 to 9 µm. The average particle diameter that is 1 µm or more is preferred since the decrease in transparency of the prosthesis can be easily suppressed. The average particle diameter that is 30 µm or less is preferred since the restorability of the dental porcelain paste can be easily enhanced.

The average particle diameter of the component (B) means the volume based average particle diameter (D50) that can be obtained through measurement using the laser diffraction scattering method, and can be measured, for example, by the method described in the examples.

The glass transition temperature of the component (B) is preferably 400 to 600°C, more preferably 420 to 580°C, and further preferably 450 to 550°C, from the standpoint of enabling the baking at a lower temperature and shortening the baking time. The softening point of the component (B) is preferably 500 to 680°C, more preferably 520 to 650°C, and further preferably 550 to 630°C, from the same standpoint. The glass transition temperature that is 400°C or more or the softening point that is 500°C or more is preferred since the component (B) can be prevented from sagging in baking. The glass transition temperature that is 600°C or less or the softening point that is 680°C or less is preferred since the baking can be performed at a low temperature, and thereby a ceramic frame formed of lithium disilicate glass ceramics or the like can be prevented from being deformed in baking at a high temperature.

The linear thermal expansion coefficient (50 to 500°C) of the component (B) may be appropriately selected depending on the material of the abutment tooth, the material of the frame, or the like, and is not particularly limited, and may be 4.0 × 10⁻⁶ to 6.0 × 10⁻⁶ /°C, for example, may be 6.1 × 10⁻⁶ to 13.5 × 10⁻⁶ /°C, and may be 6.3 × 10⁻⁶ to 12.5 × 10⁻⁶ /°C. For example, in the case where the dental porcelain paste is applied to a frame formed of zirconia as a major component, the linear thermal expansion coefficient of the component (B) is preferably 9.0 × 10⁻⁶ to 11.0 × 10⁻⁶ /°C. For example, in the case where the dental porcelain paste is applied to a frame formed of alumina as a major component, the linear thermal expansion coefficient of the component (B) is preferably 6.1 × 10⁻⁶ to 8.8 × 10⁻⁶ /°C. The linear thermal expansion coefficient can be measured by heating a specimen from room temperature to 500°C with a thermal analyzer, TMA 120 (available from Seiko Instruments Inc., temperature rise rate: 5°C/min). The linear thermal expansion coefficient can be regulated by the known method, and for example, can be regulated by the content of K₂O.

The content of the component (B) is preferably 40 to 80 parts by mass, more preferably 45 to 75 parts by mass, and further preferably 48 to 72 parts by mass, per 100 parts by mass in total of the component (B) and the component (C).

### <Alcohol (C)>

Examples of the alcohol (C) include a dihydric alcohol having a linear chain having 2 to 15 carbon atoms, such as 1,2-ethanediol, 1,2-propanediol, 1,3-propanediol, 1,2-butanediol, 1,3-butanediol, 1,4-butanediol, 2,3-butanediol, 1,2-pentanediol, 1,5-pentanediol, 2,4-pentanediol, 1,2-hexanediol, 2,5-hexanediol, ethylene glycol, diethylene glycol, triethylene glycol, tetraethylene glycol, polyethylene glycol (molecular weight: 200 to 600), propylene glycol, dipropylene glycol, and polypropylene glycol; a dihydric alcohol having a branched chain having 3 to 20 carbon atoms, such as 1-methyl-1,3-propanediol, 2-methyl-1,3-propanediol, 2-methyl-1,4-butanediol, 3-methyl-1,3-butanediol, 2-methyl-2,4-pentanediol, 3-methyl-1,5-pentanediol, 2,4-diethyl-1,5-pentanediol, and 2-ethyl-1,3-hexanediol; 3-benzyloxy-1,2-propanediol, 4-benzyloxy-1,2-butanediol, methanol, ethanol, 1-propanol, 2-propanol, isopropanol, 1-butanol, 2-butanol, 1-heptanol, 2-heptanol, 3-heptanol, 1-hexanol, 2-hexanol, 3-hexanol, 2-methyl-1-pentanol, 3-methyl-1-pentanol, 4-methyl-1-pentanol, 2-methyl-2-pentanol, 2-methyl-3-pentanol, 2,2-dimethyl-1-butanol, 2-ethyl-1-butanol, 2-phenoxyethanol, ethylene glycol monomethyl ether, ethylene glycol monoethyl ether, propylene glycol monomethyl ether, propylene glycol monoethyl ether, ethylene glycol monobutyl ether, diethylene glycol monomethyl ether, diethylene glycol monoethyl ether, propylene glycol monopropyl ether, dipropylene glycol monoethyl ether, tripropylene glycol monomethyl ether, diethylene glycol monobutyl ether, triethylene glycol monomethyl ether, glycerin, 1,2,3-butanetriol, and 1,2,6-hexanetriol.

One kind of the component (C) may be used alone, or two or more kinds thereof may be used in combination.

The component (C) is preferably an alcohol that is in a liquid state at 20°C.

The boiling point of the component (C) is preferably 100 to 300°C, more preferably 100 to 250°C, and further preferably 100 to 230°C.

The component (C) preferably contains a dihydric alcohol, more preferably contains at least one kind selected from the group consisting of 1,2-propanediol, 1,3-propanediol, 1,4-butanediol, 1,-methyl-1,3-propanediol, 2-methyl-1,3-propanediol, 2-methyl-1,4-butanediol, 3-methyl-1,3-butanediol, 2-methyl-2,4-pentanediol, 3-methyl-1,5-pentanediol, 2,4-diethyl-1,5-pentanediol, and 2-ethyl-1,3-hexanediol, and further preferably contains at least one kind selected from the group consisting of 1,2-propanediol, 1,3-propanediol, 1,4-butanediol, 3-methyl-1,5-pentanediol, and 2-ethyl-1,3-hexanediol.

The content of the dihydric alcohol in the component (C) is preferably 60 to 100% by mass, more preferably 80 to 100% by mass, and further preferably 90 to 100% by mass, and may be 100% by mass, based on the total amount of the component (C) as 100% by mass.

The content of the component (C) is not particularly limited, and is preferably 20 to 60 parts by mass, more preferably 25 to 55 parts by mass, and further preferably 28 to 52 parts by mass, per 100 parts by mass in total of the component (B) and the component (C). The content of the component (C) that is 20 parts by mass or more is preferred since the kneadability of the component (B) and the component (C) is enhanced to facilitate the formation of paste. The content of the component (C) that is 60 parts by mass or less is preferred since the amount of the glass component in the dental porcelain paste can be easily secured to facilitate the buildup of the porcelain material sufficiently.

The total content of the component (A), the component (B), and the component (C) in the dental porcelain paste is preferably 75% by mass or more, more preferably 80% by mass or more, further preferably 90% by mass or more, and still further preferably 95% by mass or more, and is 100% by mass or less, based on the dental porcelain paste as 100% by mass, from the standpoint of facilitating the effects of the present invention. In other words, in one embodiment of the dental porcelain paste, the total content of the component (A), the component (B), and the component (C) in the dental porcelain paste is preferably 75 to 100% by mass, more preferably 80 to 100% by mass, further preferably 90 to 100% by mass, and still further preferably 95 to 100% by mass, based on the dental porcelain paste as 100% by mass.

### <Optional Components>

The dental porcelain paste may further contain, in addition to the components (A) to (C), additional components (optional components) depending on necessity unless impairing the effects of the present invention. Examples of the additional components include water, a colorant, a pH modifier, a polymerization accelerator, and a polymerization initiator.

The colorant may be either an amorphous material or a crystalline material. Examples of the colorant include a pigment, such as an inorganic pigment and an opacifier, and a colorant that is decolored in baking.

Examples of the inorganic pigment include praseodymium oxide, vanadium oxide, iron oxide, nickel oxide, chromium oxide, manganese oxide, cerium oxide, a tin oxide compound (for example, a composite oxide containing tin(II) oxide or tin(IV) oxide as a component (such as vanadium-tin yellow and chromium-tin pink)), bismuth-vanadium yellow, vanadium-zirconium yellow, praseodymium yellow, cobalt blue, manganese pink, chromium-alumina pink, chromium iron zinc, titanium oxide (TiO₂), and zirconium oxide (ZrO₂).

Examples of the opacifier include zirconium silicate, tin(IV) oxide, zirconium oxide (ZrO₂), zinc oxide, titanium oxide (TiO₂), and aluminum oxide. These materials may be appropriately contained depending on the desired color tone, and in the case where white color tone is desired, it is preferred to add zirconium silicate in an amount of 5 to 20% by mass.

Examples of the colorant that is decolored in baking include an edible dye that is soluble in an organic solvent. Examples of the edible dye include an organic colorant containing two or more aromatic groups, such as Food Color Yellow No. 4 (tartrazine), Food Color Yellow No. 5 (Sunset Yellow FCF), Food Color Red No. 2 (amaranth), Food Color Red No. 102 (New Coccine), Food Color Blue No. 1 (Brilliant Blue FCF), Food Color Blue No. 2 (indigocarmine), Food Color Green No. 3 (Fast Green FCF), and Food Color Red No. 102 (New Coccine); an organic colorant containing a condensed aromatic group having xanthene as a mother nucleus (xanthene based colorant), such as Acid Red 289, Bromopyrogallol Red, Rhodamine B, Rhodamine 6G, Rhodamine 6GP, Rhodamine 3GO, Rhodamine 123, Eosin, Eosin B, Eosin Y, fluorescein, and fluorescein isothiocyanate; a cochineal colorant (carminic acid based colorant); and a betalain based colorant, such as Beat Red (major component: isobetanin and betanin), betanin, isobetanin, probetanin, and neobetanin.

Examples of the preferred production method of the dental porcelain paste as one embodiment of the present invention include a production method including at least a step of mixing the component (A), the component (B), and the component (C). The condition for mixing is not particularly limited, and the components to be mixed may be added at once, or may be added in portions. The kneader used for mixing may be an ordinary kneader. Examples thereof include a mortar, a twin-screw kneader (Twinmix), a three-screw kneader (Trimix), a kneader, and a planetary mixer. Among these, a mortar and a planetary mixer are preferably used.

In one embodiment of the present invention, the dental porcelain paste preferably contains substantially no photocurable resin. The expression "contains substantially no photocurable resin" means specifically that the photocurable resin is 3% by mass or less, preferably 1% by mass or less, more preferably 0.1% by mass or less, and further preferably 0.05% by mass or less, based on the dental porcelain paste as 100% by mass. In other words, in one embodiment of the present invention, the content of the photocurable resin is preferably 0 to 3% by mass, more preferably 0 to 1% by mass, further preferably 0 to 0.1% by mass, and still further preferably 0 to 0.05% by mass, based on the dental porcelain paste as 100% by mass. It is more preferred that the dental porcelain paste as one embodiment of the present invention contains no photocurable resin, i.e., the content thereof is 0% by mass.

The photocurable resin is a resin that is cured with an energy ray, such as an X-ray, an ultraviolet ray, and visible light. Examples of the photocurable resin include a radical polymerization type photocurable resin, such as urethane acrylate and epoxy acrylate type resins, and a cationic polymerization type photocurable resin, such as an epoxy resin.

In one embodiment of the present invention, the content of an organic polymer material having a hydrophilic group of the dental porcelain paste may be less than 0.5% by mass, may be 0.3% by mass or less, or may be 0.1% by mass or less, based on the dental porcelain paste as 100% by mass. In other words, in one embodiment of the present invention, the content of the organic polymer material having a hydrophilic group may be 0 to 0.5% by mass, may be 0 to 0.3% by mass, or may be 0 to 0.1% by mass, based on the dental porcelain paste as 100% by mass.

The organic polymer material may be, for example, an organic compound having a weight average molecular weight of 1,000 or more, and the weight average molecular weight may be, for example, a value that is obtained in terms of standard polystyrene conversion by gel permeation chromatography.

It suffices that the dental porcelain paste as one embodiment of the present invention is in the form of paste capable of being built up at the point of use, and therefore, for example, it is possible that a dental porcelain kit including a liquid composition (first composition) containing the component (A) and the component (C) and a powder composition (second composition) containing the component (B) is provided, and the dental porcelain paste is produced by mixing the first composition and the second composition immediately before use by the user. As another example, a part of the component (C) may be separately packaged, for example, a dental porcelain kit including a pre-paste containing the component (A), the component (B), and a part of the component (C), and the balance of the component (C) is provided, and the dental porcelain paste is produced by adding the balance of the component (C) to the pre-paste immediately before use by the user. In the configuration of the kits, the kinds, the contents, and the like of the components may be appropriately changed, and the optional components may be added or deleted, according to the foregoing description. In the case where the dental porcelain paste as one embodiment of the present invention is provided as the dental porcelain kit, it suffices that the requirements of the dental porcelain paste are satisfied at the time of using as the dental porcelain paste. For example, at least one composition constituting the kit may previously satisfy the requirements of the dental porcelain paste, or the formulations of the compositions may be designed to satisfy the requirements of the dental porcelain paste eventually by mixing the compositions.

### [Applications of Dental Porcelain Paste]

The dental porcelain paste as one embodiment of the present invention can be used, for example, for producing a dental prosthesis formed of ceramics, such as an inlay, an onlay, a laminate veneer, and a crown.

The frame as a target on which the dental porcelain paste as one embodiment of the present invention is built up is not particularly limited, and examples thereof include a metal frame and a ceramic frame (such as a zirconia frame).

The purpose of the dental porcelain paste is not particularly limited, and can be used, for example, as a body porcelain material (dentin color porcelain material), a cervical porcelain material, an incisal porcelain material (enamel color porcelain material), a translucent porcelain material, an opaque porcelain material, and a stained porcelain material.

For producing a dental prosthesis, the dental porcelain paste is built up and then baked. The baking temperature (baking maximum temperature) is not particularly limited, and may be appropriately changed depending on the kind of the porcelain material, the use form thereof, and the like, and the baking temperature is preferably 700°C or more, more preferably 730°C or more, and further preferably 740°C or more, and is preferably 1,050°C or less, more preferably 1,000°C or less, and further preferably 980°C or less. As described above, the lower limit values and the upper limit values described in a stepwise manner can be independently combined. For example, the baking temperature (baking maximum temperature) in one embodiment is preferably 700 to 1,050°C, more preferably 730 to 1,000°C, and further preferably 740 to 980°C. The temperature rise rate in baking to the maximum baking temperature may be appropriately changed depending on the kind of the porcelain material, and is not particularly limited, and is preferably 10 to 70°C/min, more preferably 15 to 60°C/min, and further preferably 20 to 50°C/min.

Before baking the dental porcelain paste having been built up, the dental porcelain paste may be dried, and the drying condition is not particularly limited. The dental porcelain paste having been built up may be baked in vacuum (vacuum baking) since the bubbles existing inside the glass powder (B) can be largely reduced to provide a dental prosthesis excellent in transparency, resulting in excellent aesthetic quality. The degree of vacuum in the vacuum baking is not particularly limited, and may be, for example, 750 mmHg or less. The temperature at the start of the vacuum baking is not particularly limited, and may be, for example, 550 to 700°C.

In another embodiment, the present invention provides use of a dental porcelain paste containing the component (A), the component (B), and the component (C), for the treatment of teeth (for example, aesthetic dental treatment, missing tooth treatment, prosthetic dental treatment, such as a prosthetic tooth, and dental caries treatment).

In all the embodiments described above, the kinds, the contents, and the like of the components may be appropriately changed, and the optional components may be modified, for example, added and deleted, according to the aforementioned description. In all the embodiments described above, the compositions and the characteristic values of the dental porcelain pastes may be appropriately changed and combined. Accordingly, one kind of the dental porcelain pastes described above may be used alone, or two or more kinds thereof may be used in combination, as long as exerting the effects of the present invention.

The present invention encompasses embodiments including various combinations of the aforementioned configurations within the scope of the technical concept of the present invention, as long as exerting the effects of the present invention.

### Examples

The present invention will be described more specifically with reference to examples below, but the present invention is not limited to the examples.

### [Examples 1 to 24 and Comparative Examples 1 to 4]

Dental porcelain pastes of Examples and Comparative Examples were prepared in the following manner, and evaluated for the characteristics thereof. The results are shown in Tables 1 to 3.

### <Preparation of Dental Porcelain Paste>

The components shown in Tables 1 to 3 were mixed at the contents (part by mass) shown in Tables 1 to 3 with a mortar under ordinary temperature for approximately 10 minutes, so as to prepare dental porcelain pastes. The components used were as follows.

### (Cationic Surfactant: Component (A))

· Cetylpyridinium chloride monohydrate (available from Fujifilm Wako Pure Chemical Corporation, white solid matter)
· Lauryltrimethylammonium chloride (available from Fujifilm Wako Pure Chemical Corporation, white solid matter)
· Dodecylpyridinium chloride (available from Fujifilm Wako Pure Chemical Corporation, white solid matter)
· Trimethylstearylammonium chloride (available from Tokyo Chemical Industry Co., Ltd., white solid matter)
· Dimethyloctadecylammonium chloride (available from Fujifilm Wako Pure Chemical Corporation, white solid matter)
· Benzyldimethyltetradecylammonium chloride (available from Fujifilm Wako Pure Chemical Corporation, white solid matter)
· Lauryltrimethylammonium bromide (available from Tokyo Chemical Industry Co., Ltd., white solid matter)
· Trimethylstearylammonium bromide (available from Tokyo Chemical Industry Co., Ltd., white solid matter)

### (Glass Powder: Component (B))

For the glass powder, amorphous type potassium aluminosilicate glass frit formed of SiO₂, Al₂O₃, ZnO, K₂O, Na₂O, Li₂O, CaO, MgO, Sb₂O₃, CeO₂, and BaO was pulverized with a planetary mill, so as to produce powder having an average particle diameter shown in Tables 1 to 3. For the average particle diameter, the volume based average particle diameter (D50) was measured by the laser diffraction scattering method. The measuring device used was Microtrac (registered trademark) MT3300II (available from MicrotracBEL Corporation), and water was used as the dispersion medium.

### (Alcohol: Component (C))

· 1,3-Butanediol (available from Kanto Chemical Co., Inc.)
· 1,2-Propanediol (available from Kanto Chemical Co., Inc.)
· 3-Methyl-1,5-pentanediol (available from Kuraray Co., Ltd.)
· 2-Phenoxyethanol (available from Fujifilm Wako Pure Chemical Corporation)
· Glycerin (available from Kanto Chemical Co., Inc.)

### (Additive used instead of Cationic Surfactant ("Additional component" in Table 3)

· Polyethylene glycol monostearate (available from Tokyo Chemical Industry Co., Ltd., white waxy matter)
· "Aerosil (registered trademark) R976S" (available from Nippon Aerosil Co., Ltd., hydrophobic fine particle silica, white powder)
· Xanthane gum (available from Tokyo Chemical Industry Co., Ltd., pale yellow solid matter)

### (Evaluation of Residue on Ignition of Surfactant)

The cationic surfactant, polyethylene glycol monostearate, or xanthane gum was weighed in an amount of 0.3 g into a crucible, which was placed in an electric furnace, heated to 400°C at a temperature rise rate of 20°C/min, and retained at 400°C for 1 hour. The interior of the electric furnace was the atmospheric condition. The weight of the residue obtained after heating was measured, and the residue on ignition was calculated by the following expression.

The surfactant exhibiting a residue on ignition of 0.5% or less was evaluated to have a decomposition point of 400°C or less. The decomposition point of the cationic surfactant is preferably 400°C or less.

The additives used in Comparative Examples 2 and 4 instead of the cationic surfactant were also evaluated for the residue on ignition in the same manner.

Residue on ignition (% by mass) = ((weight of specimen after heating to 400°C for 1 hour (g))/(weight of specimen before heating (g)) × 100

Weight of specimen before heating = 0.3 g

### <Evaluation of Dental Porcelain Paste>

### (1) Evaluation of Restorability after Long-term Standing

The dental porcelain pastes prepared in Examples and Comparative Examples each were weighed in an amount of 10 g into a 20 mL vessel, and allowed to stand in a thermostat chamber at 60°C for 2 week to prepare a test specimen. The test specimen after standing for 2 weeks was cooled to 23°C, and then manually agitated with a stainless steel spatula until the state of the prepared state before standing was restored. The period of time and the effort required for agitation were evaluated by the following standard.

### <Evaluation Standard>

A: The stainless steel spatula easily penetrated the test specimen, and the state before standing was restored within a period of 1 minute or less after starting agitation.
B: The stainless steel spatula penetrated the test specimen, and the state before standing was restored within a period of more than 1 minute and 3 minutes or less after starting agitation.
C: The stainless steel spatula was difficult to penetrate the test specimen, and the state before standing was restored within a period of more than 3 minutes and 10 minutes or less after starting agitation.
D: Solidification occurred to an extent that the stainless steel spatula did not penetrate the test specimen, and even though the agitation was continued for more than 10 minutes, the solidification was not reversed to fail to restore the state before standing.

### (2) Evaluation of Appearance after Baking

The dental porcelain pastes prepared in Examples and Comparative Examples each were coated to a thickness of 0.2 mm on a zirconia plate of 10 mm in length × 35 mm in width to prepare a specimen, and also to a thickness of 1.0 mm thereon to prepare a specimen, which were baked at a temperature shown in Tables 1 to 3 to provide sintered articles.

The appearance of the sintered articles was visually confirmed and evaluated by the following standard (N = 3).

### <Evaluation Standard>

A: The glass was not discolored and was transparent in both the sintered articles having a thickness of 0.2 mm and 1.0 mm.
B: The glass was not discolored and had transparency in the sintered article having a thickness of 0.2 mm, but the glass was discolored to gray with deterioration in transparency in the sintered article having a thickness of 1.0 mm.
C: The glass was discolored to gray with deterioration in transparency in both the sintered articles having a thickness of 0.2 mm and 1.0 mm.

**Table 1**

| | | | | Example | | | | | | | | | | | |
|---|---|---|---|---|---|---|---|---|---|---|---|---|---|---|---|
| | | | | 1 | 2 | 3 | 4 | 5 | 6 | 7 | 8 | 9 | 10 | 11 | 12 |
| | (A) (*1) | Cetylpyridinium chloride monohydrate | (part by mass) | 0.001 | 0.005 | | 0.2 | | 0.5 | 1.0 | 1.1 | | | | |
| | | Lauryltrimethylammonium chloride | (part by mass) | | | 0.01 | | 0.2 | | | | 1.0 | 1.0 | | 0.5 |
| | | Dodecylpyridinium chloride | (part by mass) | | | | | | | | | | | | |
| | | Lauryltrimethylammonium bromide | (part by mass) | | | | | | | | | | | 0.2 | |
| | | Trimethylstearylammonium chloride | (part by mass) | | | | | | | | | | | | |
| | | Dimethyldioctadecylammonium chloride | (part by mass) | | | | | | | | | | | | |
| Component | | Benzyldimethyltetradecylammonium chloride | (part by mass) | | | | | | | | | | | | |
| | | Trimethylstearylammonium bromide | (part by mass) | | | | | | | | | | | | |
| | (B) | Glass powder | (part by mass) | 70.0 | 70.0 | 70.0 | 70.0 | 70.0 | 70.0 | 70.0 | 70.0 | 70.0 | 70.0 | 70.0 | 50.0 |
| | (C) | 1,3-Butanediol | (part by mass) | 30.0 | 30.0 | 30.0 | 30.0 | 30.0 | 30.0 | 30.0 | 30.0 | 30.0 | | 30.0 | 50.0 |
| | | 1,2-Propanediol | (part by mass) | | | | | | | | | | 30.0 | | |
| | | 3-Methyl-1,5-pentanediol | (part by mass) | | | | | | | | | | | | |
| | | 2-Phenoxyethanol | (part by mass) | | | | | | | | | | | | |
| | | Glycerin | (part by mass) | | | | | | | | | | | | |
| Residue on ignition of component (A) | | | (%) | 0.2 | 0.2 | 0.1 | 0.2 | 0.1 | 0.2 | 0.2 | 0.2 | 0.1 | 0.1 | 0.4 | 0.1 |
| Average particle diameter of component (B) | | | (µm) | 5.0 | 5.0 | 5.0 | 5.0 | 5.0 | 5.0 | 5.0 | 5.0 | 5.0 | 5.0 | 5.0 | 5.0 |
| Evaluation (1): Restorability after long-term standing | | | (-) | C | B | A | A | A | A | A | A | A | A | A | B |
| Evaluation (2): Evaluation of appearance after baking | | | (- ) | A | A | A | A | A | A | B | B | B | B | A | A |
| Baking temperature in evaluation (2) | | | (°C) | 740 | 740 | 740 | 740 | 740 | 740 | 740 | 740 | 740 | 740 | 740 | 740 |

| | | | | | | | | | | | | | | | |
|---|---|---|---|---|---|---|---|---|---|---|---|---|---|---|---|
| (*1): Content per 100 parts by mass of component (B) | | | | | | | | | | | | | | | |

**Table 2**

| | | | | Example | | | | | | | | | | | |
|---|---|---|---|---|---|---|---|---|---|---|---|---|---|---|---|
| | | | | 13 | 14 | 15 | 16 | 17 | 18 | 19 | 20 | 21 | 22 | 23 | 24 |
| | (A) (*1) | Cetylpyridinium chloride monohydrate | (part by mass) | | | | | | | | 0.5 | 0.5 | 0.5 | 0.5 | |
| | | Lauryltrimethylammonium chloride | (part by mass) | | | | | | | | | | | | |
| | | Dodecylpyridinium chloride | (part by mass) | 0.5 | 0.5 | | | | | | | | | | 0.5 |
| | | Lauryltrimethylammonium bromide | (part by mass) | | | | | | | | | | | | |
| | | Trimethylstearylammonium chloride | (part by mass) | | | 0.2 | 0.5 | | | | | | | | |
| | | Dimethyldioctadecylammonium chloride | (part by mass) | | | | | 0.5 | | | | | | | |
| Component | | Benzyldimethyltetradecylammonium chloride | (part by mass) | | | | | | 0.5 | | | | | | |
| | | Trimethylstearylammonium bromide | (part by mass) | | | | | | | 0.2 | | | | | |
| | (B) | Glass powder | (part by mass) | 50.0 | 50.0 | 70.0 | 70.0 | 70.0 | 70.0 | 70.0 | 70.0 | 70.0 | 70.0 | 70.0 | 70.0 |
| | (C) | 1,3-Butanediol | (part by mass) | 50.0 | | 30.0 | 30.0 | | 30.0 | 30.0 | 20.0 | 20.0 | 30.0 | | |
| | | 1,2-Propanediol | (part by mass) | | | | | 30.0 | | | | | | 30.0 | 30.0 |
| | | 3-Methyl-1,5-pentanediol | (part by mass) | | 50.0 | | | | | | | | | | |
| | | 2-Phenoxyethanol | (part by mass) | | | | | | | | 10.0 | | | | |
| | | Glycerin | (part by mass) | | | | | | | | | 10.0 | | | |
| Residue on ignition of component (A) | | | (%) | 0.2 | 0.2 | 0.9 | 0.9 | 0.7 | 2.5 | 7.9 | 0.2 | 0.2 | 0.2 | 0.2 | 0.2 |
| Average particle diameter of component (B) | | | (µm) | 5.0 | 5.0 | 5.0 | 5.0 | 5.0 | 5.0 | 5.0 | 5.0 | 5.0 | 25.0 | 25.0 | 25.0 |
| Evaluation (1): Restorability after long-term standing | | | (-) | B | B | A | A | A | A | A | A | A | B | B | B |
| Evaluation (2): Evaluation of appearance after baking | | | (-) | A | A | A | B | B | B | B | B | A | A | A | A |
| Baking temperature in evaluation (2) | | | (°C) | 740 | 740 | 740 | 740 | 740 | 740 | 740 | 740 | 740 | 760 | 760 | 760 |

| | | | | | | | | | | | | | | | |
|---|---|---|---|---|---|---|---|---|---|---|---|---|---|---|---|
| (*1): Content per 100 parts by mass of component (B) | | | | | | | | | | | | | | | |

**Table 3**

| | | | | Comparative Example | | | |
|---|---|---|---|---|---|---|---|
| | | | | 1 | 2 | 3 | 4 |
| | (B) | Glass powder | (part by mass) | 70.0 | 70.0 | 70.0 | 70.0 |
| | (C) | 1,3-Butanediol | (part by mass) | 30.0 | 30.0 | 30.0 | |
| | | 1,2-Propanediol | (part by mass) | | | | 30.0 |
| Component | | 3-Methyl-1,5-pentanediol | (part by mass) | | | | |
| | | 2-Phenoxyethanol | (part by mass) | | | | |
| | | Glycerin | (part by mass) | | | | |
| | Additional component (* 1) | Polyethylene glycol monostearate | (part by mass) | | 1 | | |
| | | Aerosil R976S | (part by mass) | | | 2 | |
| | | Xanthane gum | (part by mass) | | | | 1 |
| Residue on ignition of component (A) | | | (%) | - | 0.3 | - | 9.7 |
| Average particle diameter of component (B) | | | (µm) | 5.0 | 5.0 | 5.0 | 5.0 |
| Evaluation (1): Restorability after long-term standing | | | (-) | D | D | D | B |
| Evaluation (2): Evaluation of appearance after baking | | | (-) | A | A | A | C |
| Baking temperature in evaluation (2) | | | (°C) | 740 | 740 | 740 | 740 |

| | | | | | | | |
|---|---|---|---|---|---|---|---|
| (*1): Content per 100 parts by mass of component (B) | | | | | | | |

It is understood from the results in Tables 1 and 2 that as for the dental porcelain pastes of Examples 1 to 24 containing the components (A) to (C), a sedimentation layer formed in long-term storage is not solidified, and in reusing the dental porcelain paste after long-term storage, the state immediately before producing the dental porcelain paste can be easily restored. Furthermore, it is also understood therefrom that the deterioration in appearance in baking can be suppressed.

On the other hand, it is understood from the results in Table 3 that the dental porcelain paste of Comparative Example 1 containing no component (A) is solidified in long-term storage, and the solidification cannot be reversed through agitation with a spatula to fail to restore the state before storage.

It is also understood therefrom that the dental porcelain pastes of Comparative Examples 2 and 3 containing polyethylene glycol monostearate or hydrophobic fine particle silica instead of the component (A) each are solidified in long-term storage, and the solidification cannot be reversed through agitation with a spatula to fail to restore the state before storage.

It is also understood therefrom that the dental porcelain paste of Comparative Example 4 containing xanthane gum instead of the component (A) exhibits a considerably deteriorated appearance after baking. It is estimated that the deterioration in appearance occurs due to the incomplete incineration of xanthane gum, which is a polymer compound.

### Industrial Applicability

The dental porcelain paste of the present invention can be suppressed in deterioration of the appearance in baking, and furthermore is excellent in restorability even in long-term storage.

The dental porcelain paste of the present invention is excellent in restorability in long-term storage, and thereby can restore the favorable handleability through the manual operation by the dental technician. Furthermore, the deterioration in appearance in baking can be suppressed, and thereby the color tone thereof can be accurately regulated.

Accordingly, the dental porcelain paste of the present invention can be favorably used as a dental porcelain paste that is used for prosthetic dental treatment, such as a prosthetic tooth, and is used for building up through the manual operation by the dental technician even after long-term storage.

In particular, the current situations including the increasing demand for ceramic crowns and the increasing demand for aesthetic quality are expected to lead an increased use frequency of dental porcelain, and therefore the dental porcelain paste of the present invention is useful therefor.

## Claims

1. A dental porcelain paste comprising a cationic surfactant (A), glass powder (B), and an alcohol (C).

2. The dental porcelain paste according to claim 1, wherein the dental porcelain paste has a content of the component (A) of 0.0005 to 2.0 parts by mass per 100 parts by mass of the component (B).

3. The dental porcelain paste according to claim 1 or 2, wherein the dental porcelain paste has a content of the component (C) of 20 to 60 parts by mass per 100 parts by mass in total of the component (B) and the component (C).

4. The dental porcelain paste according to any one of claims 1 to 3, wherein the dental porcelain paste has a total content of the component (A), the component (B), and the component (C) of 75% by mass or more based on the dental porcelain paste as 100% by mass.

5. The dental porcelain paste according to any one of claims 1 to 4, wherein the component (C) contains a dihydric alcohol.

6. The dental porcelain paste according to any one of claims 1 to 5, wherein the component (B) has an average particle diameter of 1 to 30 µm.

7. The dental porcelain paste according to any one of claims 1 to 6, wherein a residue on ignition of the component (A) is 8.0% or less.
